# EUROPEAN PATENT SPECIFICATION

(11) **EP 0 412 664 B1**
(45) Date of publication and mention of the grant of the patent: **17.01.1996**
(21) Application number: 90307704.8
(22) Date of filing: 13.07.1990
(51) Int. Cl.: A61M 25/10

(54) **Uterine access device with automatic cervical adjustment**
Uteruszugangsvorrichtung mit automatischer Oberflächenregelung
Dispositif d'accès utérin avec ajustement cervical automatique

(30) Priority: 10.08.1989 US 392040
(43) Date of publication of application: 13.02.1991
(73) Proprietor: C.R. BARD, INC., Murray Hill New Jersey 07974 (US)
(72) Inventor: Levine, Andy H., Boston, Massachusetts 02116 (US)
(74) Representative: Woodward, John Calvin

(56) References cited:
- EP-A- 0 088 714
- EP-A- 0 141 589
- EP-A- 0 299 158
- EP-A- 0 362 146
- US-A- 2 687 131
- US-A- 4 083 369

## Description

This invention relates to devices for accessing a uterus for manipulation or for introduction of fluids or other devices into the uterus and fallopian tubes, and to techniques for performing the uterine access.

There are a number of situations in which it is desirable to examine the uterus and fallopian tubes to evaluate female infertility. In one situation, for example, the patency of the fallopian tubes must be determined. This is usually accomplished by radiographic or laparoscopic examination.

To perform a radiographic evaluation, a radiopaque contrast liquid is injected into the uterus to enable examination under fluoroscopy of the contours of the uterus and the patency of the fallopian tube(s). This procedure is known as hysterosalpingography (HSG). When the contrast media is initially injected into the uterus, the HSG often incorrectly indicates blocked fallopian tubes. Such incorrect results are frequently due to insufficient injection pressure.

It is desirable to pressurise the uterus during injection of the contrast liquid, and a number of presently available devices attempt to do so. It is difficult to establish a seal between the uterus and the vagina, however, and leakage of the contrast liquid is a problem.

The reproductive tract can also be studied using a laparoscope, which is a tubular device inserted through an incision made in the abdomen of the patient rather than being passed through the inside of the reproductive tract. The outside of the reproductive organs and the distal openings of the fallopian tubes near the ovaries can be examined through the laparoscope. Patency of the fallopian tubes is tested by injecting a coloured dye into the uterus and observing spillage of the dye through the tubes into the abdominal cavity. Again, present injection devices may not properly seal the uterus and sufficiently high injection pressures may not be attainable. False patency test results may occur.

Particularly during laparoscopic examination, it is desirable to manipulate the uterus to permit observation of different areas of the uterus and surrounding organs. This is commonly accomplished by attempting to engage the cervix, which is the neck of the uterus, with a manipulator device. The device is inserted into the external opening of the cervix, referred to as the external os, and passed through the cervical canal into the uterus. The internal opening of the cervix is known as the internal os. Several conventional manipulator devices have a distal inflatable member which is inflated within the uterus to engage the internal os to prevent the device from pulling out of the uterus. A proximal stop is provided to prevent the device from being inserted further into the uterus.

The length of the cervical canal varies from patient to patient, however, and no one setting is appropriate for all patients. EP-A-0088714 discloses a manipulator device provided with a moveable proximal stop which must be manually adjusted in an attempt to properly seat against the external os. The process of adjusting the device to the different cervical lengths is cumbersome and may involve one or more resettings of the proximal stop to securely engage the cervix. Application of excessive force by the proximal stop may cause discomfort to the patient or undesired withdrawal of the distal member even when it is inflated.

The present invention overcomes the problems of the prior art by providing a proximal member which is expandable and formed by compliant material, such that when the proximal member is inflated with a distal portion thereof confined within a lumen such as a cervical canal, only the unconfined, more proximal portion will expand to effect a seal against the proximal end of the lumen, the spacing between the proximal and distal members and the length of the proximal member being such that when the access device is inserted into the cervical canal and the distal member is expanded and moved against the internal os thereof, the distal portion of the proximal member may be confined within the cervical canal and a more proximal portion of the proximal member may be disposed outside of the cervical canal so that when the proximal member is expanded its expanded proximal portion will effect a seal against the external os while the distal member effects a seal against the internal os.

It will be appreciated from the foregoing that the uterine access device of the invention has an element which automatically adjusts to the length of the cervical canal because when the first expandable distal member is inserted into the uterus and expanded and the second inflatable proximal member of the shaft is inserted partially through the cervical canal and inflated only the portion which is outside of the cervical canal expands so the inflated portion of the proximal member therefore is automatically positioned against the external os, and the distal and proximal members exert clamping forces toward each other to securely couple the shaft to the cervix. The variable expansion along the length of the proximal member thereby provides automatic adjustment to the actual length of the cervical canal.

Once the device of the invention has been clamped to the cervix, it can be used to manipulate the uterus, such as during laparoscopic examination of the reproductive organs, or to introduce fluids or other devices through it into the uterus. In one embodiment, the distal member is an inflatable balloon which is disposed about the shaft. The distal member sealingly engages the internal os of the cervix, and the distal and proximal members exert forces toward each other to seal the device within the cervical canal and block flow of fluid from the uterus along the exterior of the shaft. The shaft or other structure defines first and second lumens which communicate with the distal and proximal members to enable actuation of the members.

In a preferred embodiment, a third lumen is provided in the shaft through which fluids or devices can be introduced into the uterus. Alternatively, such as when the device is used only to manipulate the uterus, only the inflation lumens are provided in the shaft which is otherwise solid. Preferably, the shaft includes at least two ports through which the second lumen communicates with the proximal member, one of the ports being disposed beneath the distal portion of the proximal member and another port being disposed beneath the proximal portion. The third lumen extends from the proximal end of the shaft to its distal end, and the proximal end of the shaft extends external to the patient to serve as a handle for manipulating the uterus when the device is positioned within the cervical canal. A metal rod may be inserted into the third lumen to further stiffen the shaft.

Preferably the distal end of the shaft is inclined at an angle relative to the remainder of the shaft to facilitate insertion into the cervix and the uterus. The angle may range from 15 to 25 degrees. Further, the distal portion of the proximal member may have a wall thickness less than that of the proximal portion to preferentially induce expansion of the proximal member immediately proximal to the external os of the cervix. A preferred compliant material for the proximal member is latex. The material is sufficiently compliant that, during inflation, substantially all expansion of the proximal member occurs in the proximal portion and expansion of the distal portion is restricted by the cervical canal. Little or no dilatation of the cervical canal occurs.

A preferred technique of using such a device involves inserting the distal end of the shaft through the cervical canal to place the distal member within the uterus, expanding the distal member to increase its diameter beyond that of the cervical canal, and withdrawing the device until the expanded distal member contacts the internal os of the cervix. The proximal member then is inflated proximal to the external os of the cervix. The distal and proximal members thereby cooperate to securely couple the shaft to the uterus by automatically adjusting to the length of the cervical canal.

It will be appreciated from the foregoing that the invention provides an improved device which automatically adjusts to the specific length of the cervical canal and reliably seals the uterus to minimize leakage of dye, contrast liquid or other fluids injected into the uterus.

The device of the invention further enables higher injection pressures of fluid into the uterus and fallopian tubes and further enables an improved technique to be used for manipulating the uterus by securely and automatically gripping both ends of the cervical canal while minimizing trauma to the reproductive tract.

A preferred device of the invention will now be described, by way of example only, with reference to the accompanying drawings, in which:
Figure 1 is a schematic side view of the uterine access device of the invention;
Figure 2 is a cross-section along lines 2-2 of Figure 1; and
Figure 3 is a simplified view of the uterine access device inserted within the uterus of a patient.

A device according to this invention for accessing the uterus to manipulate it or to introduce fluids and other devices into it can be accomplished by a shaft having a first, expandable distal member which is inserted into the uterus and expanded. The shaft carries a second, inflatable proximal member which is inserted partially into the cervical canal. Upon inflation of the proximal member, only the portion which is outside of the cervical canal expands, thereby automatically adjusting to the actual length of that cervical canal. Preferably, the shaft is a hollow, elongate tube through which fluids or instruments can be introduced into the uterus and fallopian tubes.

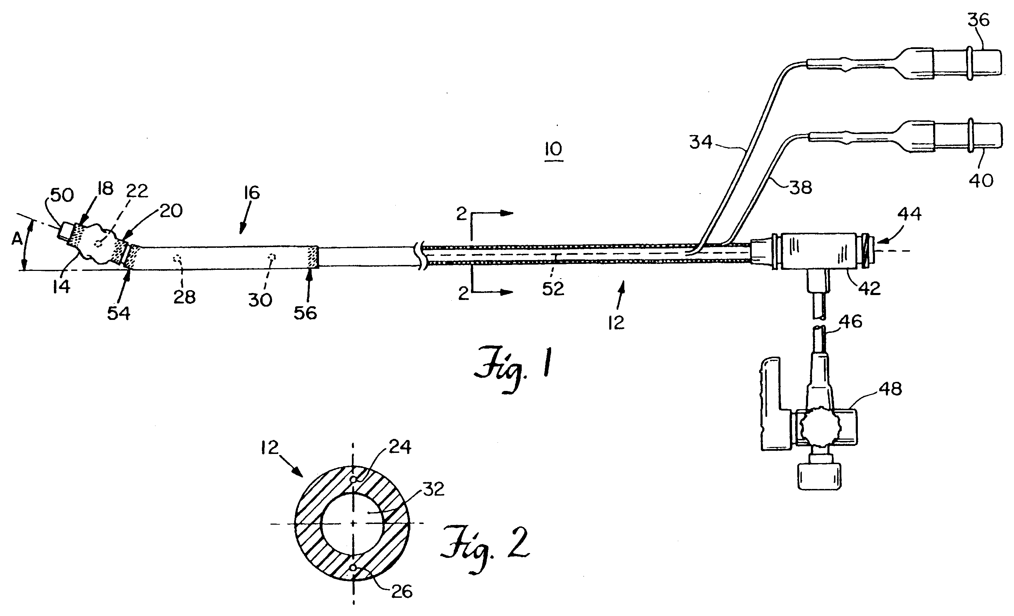

A uterine access device 10 according to this invention is shown in Fig. 1 having shaft 12 on which are disposed a first, expandable distal member 14 and a second, inflatable proximal member 16. In this construction, the distal member 14 is an inflatable balloon formed by a vinyl sleeve which is bonded at a distal region 18 and a proximal region 20 to the shaft 12. The distal member 14 is inflated through a port 22, shown in phantom, which communicates with a lumen 24 which is defined by the shaft 12 as shown in Fig. 2. The shaft 12 also defines a lumen 26 which communicates with ports 28, 30 and a large, central lumen 32 which runs the full length of the shaft 12 and terminates in a distal orifice having the same diameter as the central lumen 32. The lumens 24, 26 are preferably spaced oppositely from each other as shown. The lumen 24 is connected near its proximal end to a tube 34 which terminates in a self sealing air valve 36. Similarly, the lumen 26 is connected near its proximal end with a tube 38 and a air valve 40.

Preferably, the shaft 12 is a vinyl or polycarbonate tube formed by extrusion to have a length of 10.75 inch (27.3 cm), an outer diameter of 0.157 inch (4 mm) and an inner diameter of 0.080 inch (2 mm). Lumens 24, 26 are preferably 0.010 inch (0.25 mm) in diameter. A valve device 42 is connected to the proximal end of the shaft 12 to enable instruments or other devices to be inserted through the lumen 32 while maintaining a seal to prevent the escape of fluids through opening 44 when contrast liquids or other fluids are injected into the uterus. The seal is maintained by a gasket which closes around the device, such as found in US-A-4,424,833 by Spector et al. The device 42 further includes an injection port accessed through tube 46 and stopcock 48. Dye, contrast liquids, or other fluids can thereby be injected into the central lumen 32 to be discharged through the distal orifice at the distal end 50 of the shaft 12.

The distal end 50 of the shaft is rounded to avoid trauma to the cervical canal and the uterus, and a distal portion of the shaft 12 is preferably inclined relative to the longitudinal axis 52 of the shaft 12. The inclination of the distal portion provides easier location of and insertion into the cervical canal because the cervix and uterus are inclined relative to the vagina. Also, the inclination of the distal portion minimizes trauma to the cervix and uterus. The inclination is represented by angle A which may range from 0-30° and is preferably 15-25°. Angle A in this construction is 20 degrees, and the length of the inclined distal portion is 0.6 inch (15.2 mm).

Preferably, the second, proximal balloon 16 is more compliant than the first, distal balloon 14. Rather than vinyl or polyurethane, which is moderately compliant and suitable for the distal balloon 14, the proximal balloon 16 is preferably formed of a latex material. The proximal balloon 16 is of sufficient length so that a distal portion remains within the cervical canal while the remaining proximal portion inflates, as described in more detail below. The proximal balloon 16 is preferably at least 1.5 inch (38 mm) in length, and in this construction is 2.0 inch (51 mm) in length and secured to the shaft 12 by bonds 54, 56 which are each approximately 0.08 inch (2 mm) in length. Both the distal and proximal balloons can be bonded with a cyanoacrylate such as Loctite 495 available from Loctite Corporation. Alternatively, an epoxy such as FDA 2 available from Tra-Con, Inc. can be used. In another construction, the proximal and distal ends of the proximal balloon 16 are wrapped with a suture thread which is tied and/or coated with adhesive to secure the thread about the balloon 16.

It is desirable for the wall thickness of the proximal balloon 16 to increase from the distal bond 54 toward the proximal bond 56 to preferentially induce expansion immediately proximal to the external os. In this construction, the proximal balloon 16 has a wall of 0.010 inch (0.25 mm) at the distal end ranging proximally to 0.012 inch (0.30 mm) at the proximal end. This is accomplished during manufacture by dip coating the balloon with a mandrel which is held in a vertical position, thereby allowing gravity to draw slightly more material toward the downward end, which becomes the proximal end of the balloon. By comparison, the distal balloon 14 when fabricated of vinyl has a wall thickness of 0.015 inch (0.38 mm) and length of 0.5 inch (12.7 mm).

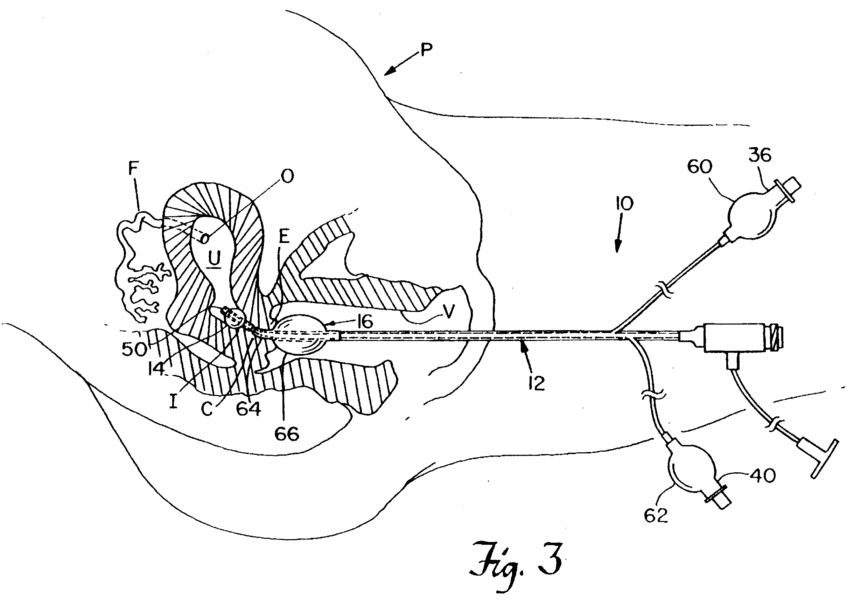

The operation of the uterine access device 10 according to the invention is shown in Fig. 3 in relation to a female patient P. After the vagina V is spread using a standard vaginal speculum (not shown), the distal end of the shaft 12 is inserted through the external os E of the cervix C. The distal balloon 14 is maintained in a deflated condition while it is inserted through the cervical canal and into the uterus U past the internal os I of the cervix C. The distal balloon 14 is then inflated to a diameter of 12-15 mm through the air valve 36 to 3-5 psig (155-260 mm Hg). Inflation of the distal balloon 14 is indicated to the operator by a vinyl pilot balloon 60. The pilot balloon in one construction is 1.3 inch (33 mm) in length, has a wall thickness of 0.040 inch, (1.0 mm) and inflates to a diameter of 0.44 inch (11.1 mm). The proximal portion of the shaft 12 is then grasped by the operator and gently pulled proximally until resistance is met, indicating that the inflated distal balloon 14 is seated against the internal os I. The proximal balloon 16 is then inflated through the air valve 40, having a pilot balloon 62, to a pressure approximately equal to that of the distal balloon 14. The pilot balloon 62 indicates whether the proximal balloon 16 is pressurized.

Variation in length of the cervical canal is accommodated by the variable expansion along the length of the proximal balloon 16. The cervical canal is commonly 2-3 cm in length, and variations of even 1 or 2 mm can affect the quality of the seal established by standard uterine manipulative or injector devices. The uterine access device 10 according to the invention, however, automatically compensates for the variation in length because the compliant material of the balloon 16 only expands where it is not restricted by the cervical canal. Little or no dilation of the cervical canal is made by the distal portion 64, while the proximal portion 66 expands to approximately 1 inch in diameter. The distal balloon 14 and the proximal balloon 16 exert opposing forces toward each other and thereby cooperate to securely couple the device 10 to the cervix and, therefore, to the uterus U.

It is desirable for the shaft 12 to be relatively rigid when uterine manipulation is required. A central lumen communicating with the interior may not be necessary when only uterine manipulation is to be performed. When it is simply desired to introduce devices or fluids into the uterus or fallopian tubes, rigidity is not required and the shaft can be more flexible. Additionally, some or all of the proximal portion of the shaft can be curved upwardly in the same direction as the inclination of the distal portion to provide a more comfortable access angle for the operator. A proximal curvature is also useful to indicate the orientation of the distal tip 50. After insertion into the uterus, the distal tip 50 can be turned laterally to facilitate access to one of the fallopian tubes. The left fallopian tube F is shown with opening O into the uterus U.

The shaft 12 may be adjustably stiffened by removably installing a metal rod within the central lumen 32, as indicated in phantom by dashed line 52. In one construction the rod is a stainless steel rod having a diameter of 0.070 inch (1.8 mm) and carrying a luer fitting which mates with the proximal end of the device 42 to secure the rod in place. The outer diameter of the rod is small enough to allow contrast liquid or other fluid to pass alongside it within the central lumen 32. The rod may be sufficiently long and flexible to extend to the distal end 50 of shaft 12, or may extend only to the distal end of the proximal balloon 16. It is desirable for the rod to extend beyond the proximal portion 66 so that the rod lies at least partially within the cervix C.

It is preferable for the shaft 12 to define at least two ports through which fluid can be introduced into the proximal balloon 16, the ports being formed by skives to provide an opening of 0.063 inch (1.6 mm). The use of at least two longitudinally spaced ports is particularly important during deflation, when the port which lies beneath the distal portion 64 can become occluded by the balloon material before all of the fluid in the proximal portion 66 is evacuated.

While the first, distal member 14 is described above as also being an inflatable balloon, this is not a limitation of the invention. The distal member 14 can be accomplished by any device which is expandable to prevent inadvertent withdrawal of the shaft 12 from the uterus U. For example, a slitted tube which expands when compressed, referred to as a mallecot, can be used. A mallecot is found for example on the Bardex Foley catheter model no. 086, a urinary catheter available from Bard Urological Division of C.R. Bard, Inc. A pull wire then passes through the lumen 24 to activate the mallecot. An expandable member having an integral proximal portion, such as an inflatable balloon, is preferred when contrast liquid or other fluid is injected into the uterus.

From the foregoing it will be appreciated that the invention provides a self adjusting device and technique for securely gripping a patient's cervix in a manner that provides a superior seal for HSG as well as a firm grip for uterine manipulation.

Various changes and modifications to the embodiments shown in the drawings and described above may be made within the scope of the invention as claimed. Therefore, it is intended that all matter contained in the above description and shown in the accompanying drawings shall be interpreted in an illustrative and not limiting sense.

## Claims

1. A uterine access device for insertion through an adult female cervical canal having an internal os and an external os, the access device comprising:
an elongate shaft (12) having a proximal end and a distal end;
a first expandable distal member (14) carried by said shaft near the distal end thereof; and
a second proximal member (16) mounted on the shaft proximal to the distal member (14) to engage the external os characterised in that the proximal member (16) is expandable and formed by compliant material, such that, when the proximal member (16) is inflated with a distal portion thereof confined within a lumen such as a cervical canal, only the unconfined, more proximal portion will expand to effect a seal against the proximal end of the lumen;
the spacing between the proximal and distal members (14,16) and the length of the proximal member (16) being such that when the access device is inserted into the cervical canal and the distal member (14) is expanded and moved against the internal os thereof, the distal portion of the proximal member (16) may be confined within the cervical canal and a more proximal portion of the proximal member (16) may be disposed outside of the cervical canal so that when the proximal member (16) is expanded, its expanded proximal portion will effect a seal against the external os while the distal member (14) effects a seal against the internal os.

2. The device as claimed in claim 1 characterised in that said distal member (14) is inflatable and encircles said shaft.

3. A device as claimed in claim 1 or claim 2 characterised in that the spacing between the proximal and distal members (14,16) is no greater than 3mm so that when said distal member is expanded distal to the cervical canal and sealingly engages the internal os of the cervix, said distal and proximal members will exert forces toward each other to seal the device within the cervical canal and block flow of fluid from the uterus along the exterior of said shaft.

4. A device as claimed in any preceding claim characterised by means defining a first lumen (24) having a proximal end and a distal end communicating with said distal member (14).

5. The device as claimed in any preceding claim characterised by means defining a second lumen (26) having a proximal end accessible external to the patient when the device is inserted and a distal end communicating with said proximal member (16).

6. The device as claimed in claim 5 characterised in that said shaft (12) defines at least two ports (28,30) through which said second lumen (26) communicates with said proximal member (16).

7. The device as claimed in claim 6 characterised in that one of said ports (28,30) is longitudinally spaced from the other of said ports.

8. The device as claimed in claim 6 or claim 7 characterised in that one of said ports (28) is disposed beneath the distal portion of said proximal member (16) and another of said ports (30) is disposed beneath the proximal portion.

9. The device as claimed in any preceding claim characterised in that the shaft (12) includes a third lumen (32) through which fluids or devices can be introduced into the uterus of the patient.

10. The device as claimed in claim 9 characterised in that the third lumen (32) extends from the proximal end of said shaft (12) to its distal end.

11. The device as claimed in claim 9 or claim 10 characterised by means (52) removably insertable into said third lumen (32) for selectively stiffening the shaft (12).

12. The device as claimed in claim 11 wherein said stiffening means comprises a metal rod (52) insertable through the proximal end of the shaft (12) to extend within said third lumen (32) beyond the proximal portion of the proximal member (16).

13. The device as claimed in any preceding claim characterised in that the proximal end of said shaft (12) serves as a handle for manipulating the uterus.

14. The device as claimed in any preceding claim characterised in that the proximal member (16) is formed of latex.

15. The device as claimed in claim 14 characterised in that the proximal member (16) is at least 38mm (1.5 inch) in length.

16. A device as claimed in any preceding claim characterised in that the distal end of the shaft (12) is inclined at an angle relative to the remainder of said shaft to facilitate insertion into the uterus.

17. The device as claimed in claim 16 characterised in that said angle is between 15 and 25 degrees.

## Patentansprüche

1. Uterus-Zugangsvorrichtung für die Einführung durch einen Gebärmutterhalskanal einer erwachsenen Frau, der einen inneren Muttermund und einem äußeren Muttermund aufweist, wobei die Zugangsvorrichtung umfaßt:
einen länglichen Schaft (12) mit einem proximalen Ende und einem distalen Ende;
ein erstes ausdehnungsfähiges, distales Organ (14), das von dem Schaft nahe dessen distalem Ende getragen wird; und
ein zweites, proximales Organ (16), das an dem Schaft proximal zum distalen Organ (14) angebracht ist, um mit dem äußeren Muttermund in Eingriff zu gelangen,
dadurch gekennzeichnet, daß das proximale Organ (16) ausdehnungsfähig ist und derart aus einem nachgiebigen Material gebildet ist, daß, wenn das proximale Organ (16) aufgeblasen wird, während ein distaler Abschnitt desselben in einem Lumen wie einem Gebärmutterhalskanal eingeschlossen ist, sich nur der nicht eingeschlossene, proximalere Abschnitt ausdehnen wird, um eine Abdichtung gegen das proximale Ende des Lumens zu bewirken;
wobei der Abstand zwischen dem proximalen (16) und dem distalen Organ (14) und die Länge des proximalen Organs (16) derart sind, daß, wenn die Zugangsvorrichtung in den Gebärmutterhalskanal eingeführt wird und das distale Organ (14) ausgedehnt und gegen dessen inneren Muttermund bewegt wird, der distale Abschnitt des proximalen Organs (16) in dem Gebärmutterhalskanal eingeschlossen werden kann und ein proximalerer Abschnitt des proximalen Organs (16) außerhalb des Gebärmutterhalskanals angeordnet werden kann, so daß bei Ausdehnung des proximalen Organs (16) sein ausgedehnter proximaler Abschnitt eine Abdichtung gegen den äußeren Muttermund bewirken wird, während das distale Organ (14) eine Abdichtung gegen den inneren Muttermund bewirkt.

2. Vorrichtung nach Anspruch 1, dadurch gekennzeichnet, daß das distale Organ (14) aufblasbar ist und den Schaft ringförmig umgibt.

3. Vorrichtung nach Anspruch 1 oder Anspruch 2, dadurch gekennzeichnet, daß der Abstand zwischen dem proximalen Organ (16) und dem distalen Organ (14) höchstens 3 mm beträgt, so daß, wenn das distale Organ distal zum Gebärmutterhalskanal ausgedehnt wird und dichtend mit dem inneren Muttermund des Gebärmutterhalses in Eingriff gelangt, die distalen und proximalen Organe aufeinander zu gerichtete Kräfte ausüben werden, um die Vorrichtung in dem Gebärmutterhals abzudichten und den Fluß von Fluid aus dem Uterus entlang der Außenseite des Schafts zu hemmen.

4. Vorrichtung nach irgendeinem der vorhergehenden Ansprüche, gekennzeichnet durch Mittel, die ein erstes Lumen (24) definieren, welches ein proximales Ende und ein mit dem distalen Organ (14) in Verbindung stehendes distales Ende aufweist.

5. Vorrichtung nach irgendeinem der vorhergehenden Ansprüche, gekennzeichnet durch Mittel, die ein zweites Lumen (26) definieren, welches ein proximales Ende, das außerhalb der Patientin zugänglich ist, wenn die Vorrichtung eingesetzt ist, und ein mit dem proximalen Organ (16) in Verbindung stehendes distales Ende aufweist.

6. Vorrichtung nach Anspruch 5, dadurch gekennzeichnet, daß der Schaft (12) mindestens zwei Öffnungen (28, 30) definiert, durch die das zweite Lumen (26) mit dem proximalen Organ (16) in Verbindung steht.

7. Vorrichtung nach Anspruch 6, dadurch gekennzeichnet, daß eine dieser Öffnungen (28, 30) in Längsrichtung von der anderen Öffnung beabstandet ist.

8. Vorrichtung nach Anspruch 6 oder Anspruch 7, dadurch gekennzeichnet, daß eine der Öffnungen (28) unterhalb des distalen Abschnitts des proximalen Organs (16) und eine andere der Öffnungen (30) unterhalb des proximalen Abschnitts angeordnet ist.

9. Vorrichtung nach irgendeinem der vorhergehenden Ansprüche, dadurch gekennzeichnet, daß der Schaft (12) ein drittes Lumen (32) einschließt, durch das Fluide oder Vorrichtungen in den Uterus der Patientin eingeführt werden können.

10. Vorrichtung nach Anspruch 9, dadurch gekennzeichnet, daß sich das dritte Lumen (32) von dem proximalen Ende des Schafts (12) zu seinem distalen Ende erstreckt.

11. Vorrichtung nach Anspruch 9 oder Anspruch 10, gekennzeichnet durch Mittel (52), die zwecks selektiver Aussteifung des Schafts (12) entfernbar in das dritte Lumen (32) eingeführt werden können.

12. Vorrichtung nach Anspruch 11, worin die Aussteifungsmittel einen Metallstab (52) umfassen, der durch das proximale Ende des Schafts (12) eingeführt werden kann, um sich innerhalb des dritten Lumens (32) bis hinter den proximalen Abschnitt des proximalen Organs (16) zu erstrecken.

13. Vorrichtung nach irgendeinem der vorhergehenden Ansprüche, dadurch gekennzeichnet, daß das proximale Ende des Schafts (12) als Griff zum Manipulieren des Uterus dient.

14. Vorrichtung nach irgendeinem der vorhergehenden Ansprüche, dadurch gekennzeichnet, daß das proximale Organ (16) aus Latex ausgebildet ist.

15. Vorrichtung nach Anspruch 14, dadurch gekennzeichnet, daß das proximale Organ (16) mindestens 38 mm (1,5 Inch) lang ist.

16. Vorrichtung nach irgendeinem der vorhergehenden Ansprüche, dadurch gekennzeichnet, daß das distale Ende des Schafts (12) in bezug auf den übrigen Schaft in einem Winkel abgebogen ist, um die Einführung in den Uterus zu erleichtern.

17. Vorrichtung nach Anspruch 16, dadurch gekennzeichnet, daß der Winkel zwischen 15 und 25 Grad beträgt.

## Revendications

1. Dispositif d'accès utérin destiné à être inséré dans le canal cervical d'un patient de sexe féminin ayant une embouchure interne et une embouchure externe, ledit dispositif d'accès comprenant :
- un arbre allongé (12) ayant une extrémité proximale et une extrémité distale,
- un premier élément dilatable distal (14) porté par cet arbre au voisinage de son extrémité distale, et
- un second élément dilatable proximal (16), monté sur l'arbre proximalement par rapport à l'élément distal (14) de manière à recontrer l'embouchure externe,
caractérisé en ce que
l'élément proximal (16) est dilatable et formé d'un matériau souple de telle manière que quand l'élément proximal (16) est gonflé alors qu'une portion distale est enfermée dans une lumière telle qu'un canal cervical, seule la portion non enfermée plus proximale se dilate de manière à créer une étanchéité vis-à-vis de l'extrémité proximale de la lumière,
l'espacement entre les éléments proximal et distal (14, 16) et la longueur de l'élément proximal (16) étant tels que quand le dispositif d'accès est inséré dans le canal cervical et l'élément distal (14) est dilaté et déplacé contre son embouchure interne, la portion distale de l'élément proximal (16) peut être enfermée à l'intérieur du canal cervical et une portion plus proximale de l'élément proximal (16) peut être situé à l'extérieur du canal cervical de sorte que quand l'élément proximal (16) est dilaté, sa portion proximale dilatée crée une étanchéité vis-à-vis de l'embouchure externe, tandis que l'élément distal (14) crée une étanchéité vis-à-vis de l'embouchure interne.

2. Dispositif selon la revendication 1, caractérisé en ce que l'élément distal (14) est gonflable et encercle ledit arbre.

3. Dispositif selon la revendication 1 ou la revendication 2, caractérisé en ce que l'espacement entre les éléments distal (14) et proximal (16) n'est pas supérieur à 3 mm, de sorte que, quand ledit élément distal est dilaté distalement par rapport au canal cervical et rencontre de façon étanche l'embouchure interne du col, lesdits éléments distal et proximal exercent des forces l'un vers l'autre de manière à rendre étanche le dispositif vis-à-vis du canal cervical et à bloquer l'écoulement du fluide provenant de l'utérus le long de l'extérieur dudit arbre.

4. Dispositif selon l'une quelconque des revendications précédentes, caractérisé par des moyens définissant une première lumière (24) ayant une extrémité proximale et une extrémité distale communiquant avec ledit élément distal (14).

5. Dispositif selon l'une quelconque des revendications précédentes, caractérisé par des moyens définissant une seconde lumière (26) ayant une extrémité proximale accessible depuis l'extérieur du patient quand le dispositif est inséré et une extrémité distale communiquant avec ledit élément proximal (16).

6. Dispositif selon la revendication 5, caractérisé en ce que ledit arbre (12) comporte au moins deux orifices (22, 30) à travers lesquels ladite seconde lumière (26) communique avec ledit élément proximal (16).

7. Dispositif selon la revendication 6, caractérisé en ce que l'un de ces orifices (28, 30) est espacé longitudinalement de l'autre de ces orifices.

8. Dispositif selon la revendication 6 ou la revendication 7, caractérisé en ce que l'un desdits orifices (28) est situé au-dessous de la portion distale dudit élément proximal (16) et l'autre de ces orifices est situé au-dessous de la portion proximale.

9. Dispositif selon l'une quelconque des revendications précédentes, caractérisé en ce que l'arbre (12) comporte une troisième lumière (32) à travers laquelle des fluides ou des dispositifs peuvent être introduits dans l'utérus de la patiente.

10. Dispositif selon la revendication 9, caractérisé en ce que la troisième lumière (32) s'étend de l'extrémité proximale à l'extrémité distale dudit arbre (12).

11. Dispositif selon la revendication 9 ou la revendication 10, caractérisé par des moyens (52) insérables et extractibles dans ladite troisième lumière (32) en vue de rigidifier sélectivement l'arbre (12).

12. Dispositif selon la revendication 11, dans lequel lesdits moyens de rigidification consistent en une tige métallique (52) insérable par l'extrémité proximale de l'arbre (12) de manière à s'étendre à l'intérieur de ladite troisième lumière (32) au-delà de la portion proximale de l'élément proximal (16).

13. Dispositif selon l'une quelconque des revendications précédentes, caractérisé en ce que l'extrémité proximale dudit arbre (12) sert de prise pour la manipulation de l'utérus.

14. Dispositif selon l'une quelconque des revendications précédentes, caractérisé en ce que l'élément proximal (16) est réalisé en latex.

15. Dispositif selon la revendication 14, caractérisé en ce que l'élément proximal (16) a au moins 38 mm de long.

16. Dispositif selon l'une quelconque des revendications précédentes, caractérisé en ce que l'extrémité distale de l'arbre (12) est inclinée angulairement par rapport au reste dudit arbre pour faciliter l'insertion dans l'utérus.

17. Dispositif selon la revendication 16, caractérisé en ce que ledit angle est compris entre 15 et 25 degrés.
